(19) Europäisches Patentamt / European Patent Office / Office européen des brevets

(11) **EP 2 979 612 A1**

(12) **EUROPEAN PATENT APPLICATION**
published in accordance with Art. 153(4) EPC

(43) Date of publication:
**03.02.2016 Bulletin 2016/05**

(51) Int Cl.:
**A61B 1/00** *(2006.01)*    **G02B 23/24** *(2006.01)*

(21) Application number: **14773982.5**

(22) Date of filing: **19.03.2014**

(86) International application number:
**PCT/JP2014/057484**

(87) International publication number:
**WO 2014/156863 (02.10.2014 Gazette 2014/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **26.03.2013   JP 2013064221**

(71) Applicant: **Olympus Corporation**
**Shibuya-ku**
**Tokyo 151-0072 (JP)**

(72) Inventors:
• **TOJO, Ryo**
**Hachioji-shi**
**Tokyo 192-8512 (JP)**

• **HANE, Jun**
**Hachioji-shi**
**Tokyo 192-8512 (JP)**
• **FUJITA, Hiromasa**
**Hachioji-shi**
**Tokyo 192-8512 (JP)**

(74) Representative: **Winter, Brandl, Fürniss, Hübner, Röss, Kaiser, Polte - Partnerschaft mbB**
**Patent- und Rechtsanwaltskanzlei**
**Bavariaring 10**
**80336 München (DE)**

(54) **INSERTION DEVICE**

(57)    An insertion apparatus includes an inserting section (112) that is to be inserted into an insertion target (P), main body (102) as a reference unit that is disposed outside the insertion target (P), a connection cable (108) that includes a movable section configured to move continuously and connects the inserting section (112) and the main body(102), and a first shape sensor (204) that detects a shape of the cable with respect to the main body (102) by detecting a movable amount of the connection cable (108).

FIG. 2

**Description**

Technical Field

The present invention relates to an insertion apparatus.

Background Art

Endoscope systems are a known example of insertion apparatuses that each includes an inserting section to be inserted into a given insertion target. For example, an endoscope system of Jpn. Pat. Appln. KOKAI Publication No. 2003-052614 includes an inserting section in which a plurality of flexible optical fibers for sensing bends are placed extending substantially over the entire length thereof. Each of the optical fibers for sensing bends includes a plurality of bending sensors and is configured to change light transmittance to the bending sensors in accordance with the curvature thereof. Such an endoscope system of Jpn. Pat. Appln. KOKAI Publication No. 2003-052614 detects the bending state of the optical fibers on the basis of an output from the respective bending sensors that are disposed in the optical fibers for sensing bends, and causes a display to display the detected bending state as the bending state of the inserting section.

Summary of Invention

Jpn. Pat. Appln. KOKAI Publication No. 2003-052614 has proposed displaying the shape of the inserting section. Seeing the shape, however, does not always mean seeing the position of the inserting section in space in which the endoscope system is used.
The present invention is made in view of the above circumstances. It is an object of the invention to provide an insertion apparatus with which an operator can recognize the shape and the position of an inserting section.
According to an aspect of the invention, an insertion apparatus comprises : an inserting section that is to be inserted into an insertion target; a reference unit that is disposed outside the insertion target; a connecting member that includes a movable section configured to move continuously and connects the inserting section and the reference unit; and a first shape sensor that detects a shape of the connecting member with respect to the reference unit by detecting a movable amount of the connecting member.
The present invention provides an insertion apparatus with which an operator can recognize the shape and the position of an inserting section.

Brief Description of Drawings

FIG. 1 is a diagram illustrating an entire configuration of an insertion apparatus according to embodiments of the present invention.
FIG. 2 is a diagram illustrating a configuration of a flexible endoscope as an example of an insertion apparatus according to a first embodiment of the present invention.
FIG. 3A is a first diagram illustrating a curve detecting section.
FIG. 3B is a second diagram illustrating a curve detecting section.
FIG. 3C is a third diagram illustrating a curve detecting section.
FIG. 4 is a diagram illustrating a structure in which optical fibers are installed in an endoscope.
FIG. 5 is a diagram illustrating a display example of information about the shape of an inserting section.
FIG. 6 is a diagram illustrating a configuration of a flexible endoscope according to a modification in which a first shape sensor and a second shape sensor are integrated.
FIG. 7A is a diagram illustrating a display example where an image of the interior of an insertion target as well as an image of the shape of an inserting section is displayed.
FIG. 7B is a diagram illustrating a display example where a reference position of the insertion target can be determined.
FIG. 8 is a diagram illustrating a configuration of a flexible endoscope using an antenna and a coil serving as an insertion target position detector as an example of an insertion apparatus according to a second embodiment of the present invention.
FIG. 9 is a diagram illustrating an arrangement example where a plurality of coils are disposed.
FIG. 10 is a diagram illustrating a configuration of a flexible endoscope according to a modification in which a shape sensor for detecting an insertion target position is used.
FIG. 11 is a diagram illustrating a configuration of a rigid endoscope as another example of an insertion apparatus.

Description of Embodiments

The following describes embodiments of the present invention with reference to the accompanying drawings.

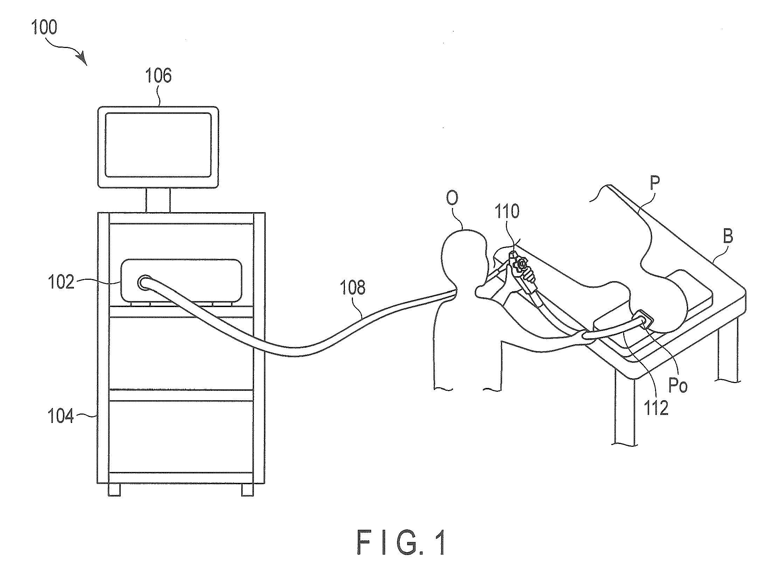

FIG. 1 is a diagram illustrating an entire configuration of an insertion apparatus according to embodiments of the present invention. FIG. 1 exemplifies a flexible endoscope serving as an insertion apparatus. The insertion apparatus according to the present embodiment is, however, not limited to a flexible endoscope and may be an insertion apparatus that includes an inserting section to be inserted into an insertion target, such as a rigid endoscope, a catheter, and a treatment tool.
An insertion apparatus 100 includes a main body 102, a rack 104, a display 106, and an endoscope. As illustrated in FIG. 1, the main body 102 is placed in the rack 104 and is configured to maintain a relative position with an insertion target P while the insertion apparatus 100 is being used. The display 106 is also placed on the rack 104. The main body 102 and the display 106 are connected to each other via a connection cable not illustrated so as to communicate with each other.

The endoscope is connected to the main body 102. "The endoscope" in the present embodiment includes a connection cable 108, an operating section 110, and an inserting section 112, but does not include the main body 102. When using the insertion apparatus 100, an operator O operates the endoscope while holding the operating section 110 and the inserting section 112; meanwhile, the insertion target (a patient, for example) P of the insertion apparatus 100 is laid on a bed B so as not to move while the insertion apparatus 100 is being used. The operator O inserts the inserting section 112 into the insertion target P from an entrance Po, for example, a mouth, and observes the interior of the insertion target P.

<First Embodiment>

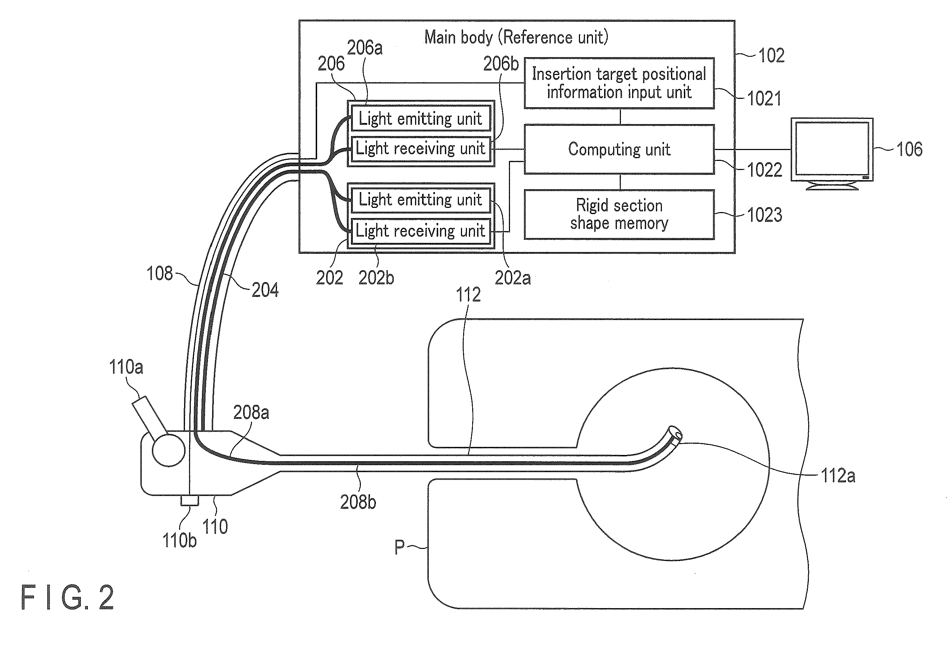

The following describes a first embodiment. FIG. 2 is a diagram illustrating a configuration of a flexible endoscope as an example of an insertion apparatus 100 according to the first embodiment of the present invention. The following describes a main body 102 and an endoscope in this order.

[Main Body]

The main body 102 includes an insertion target positional information input unit 1021, a computing unit 1022, and a rigid section shape memory 1023. The main body 102 further includes light receiving/emitting units 202 and 206 for two types of shape sensors to be described in detail later. The main body 102 is a reference unit that computes a reference position for detecting the shape and the position of the inserting section 112 of the endoscope. The main body 102 is thus configured to maintain a relative position with the insertion target P while the endoscope is operated. For example, when the insertion target P is laid on the bed B, the main body 102 is placed in the rack 104 so that the main body 102 will not move.

The insertion target positional information input unit 1021 acquires a signal from an insertion target reference position input section 110b as association information about the insertion target reference position, and inputs the signal to the computing unit 1022. The computing unit 1022 computes the shape and the position of the inserting section 112 with respect to the reference position. The rigid section shape memory 1023 stores shape information on the operating section 110, which is a rigid section of the endoscope. The rigid section shape memory 1023 is a well-known storage medium such as a flash memory and a hard disk drive.

[Endoscope]

As described above, the endoscope includes the connection cable 108, the operating section 110, and the inserting section 112. The endoscope further includes a first shape sensor 204 and second shape sensors 208a and 208b. The first shape sensor 204 is so disposed as to pass through the interior of the connection cable 108. The second shape sensors 208a and 208b are so disposed as to pass through the interior of the connection cable 108, the operating section 110, and the inserting section 112.

The connection cable 108 is a connecting member that connects the main body 102 to the operating section 110 electrically and optically. The connection cable 108 is a movable section by which the operating section 110 and the inserting section 112 can be moved. Specifically, the connection cable 108 is made of a flexible member and can change its shape continuously. In the meantime, a connecting section between the main body 102 and the connection cable 108 is fixed in place and fixed so as not to rotate about the longitudinal axis of the connection cable 108. An articulated mechanism using a ball joint, for example, may be used to constitute the movable section of the connection cable 108.

The operating section 110 serving as a connecting member in conjunction with the connection cable 108 includes a curve control lever 110a and the insertion target reference position input section 110b. The operating section 110 is configured so as not to be movable unlike the connection cable 108.

The curve control lever 110a is a control lever for the operator O to control the curved shape of the distal end of the inserting section 112. The operator O can curve the distal end of the inserting section 112 by controlling the curve control lever 110a.

The insertion target reference position input section 110b is, for example, a switch operated by the operator O. The insertion target reference position input section 110b is pressed by the operator O when the distal end of the inserting section 112 reaches a predetermined reference position for the insertion target P. In response to the insertion target reference position input section 110b being pressed, a signal to indicate that the insertion target reference position input section 110b is pressed is input to the insertion target positional information input unit 1021 in the main body 102. The reference position is the entrance Po to an opening (a mouth, for example) of the insertion target P, for example, but is not limited to this. The reference position may be determined optionally by the operator O. However, the reference position is preferably set at a position the operator can recognize easily, such as an entrance to an opening of the insertion target P and a bifurcation inside the insertion target P.

The inserting section 112 is configured movably similarly to the connection cable. The inserting section 112 includes an imaging section 112a at its distal end. The imaging section 112a captures an image of the interior of the insertion target P and outputs an electric signal (imaging signal) in accordance with the image of the interior of the insertion target P.

The first shape sensor 204 and the second shape sensors 208a and 208b are optical fiber curved shape sensors, for example. The optical fiber curved shape sensors each include optical fibers having curve detecting sections disposed at various locations thereon.

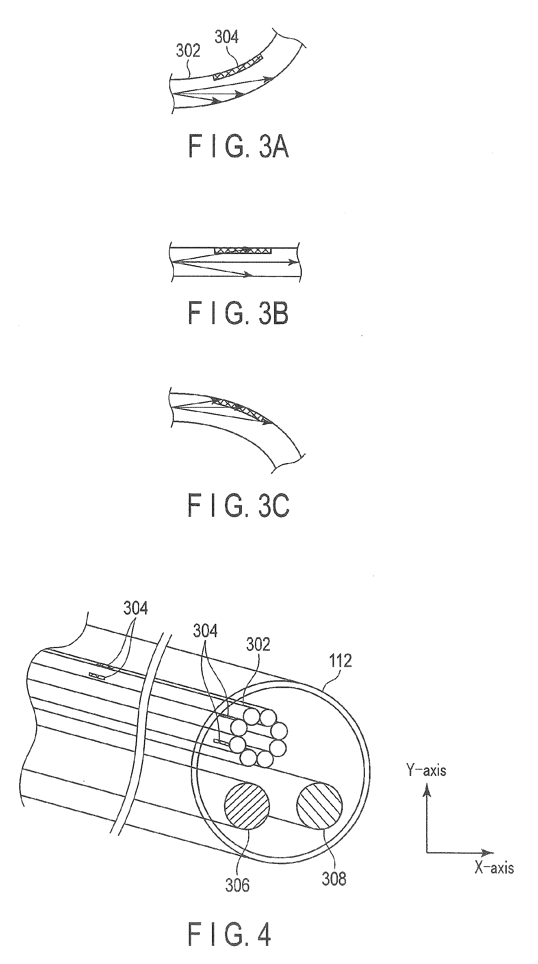

FIG. 3A to FIG. 3C are diagrams illustrating the curve detecting section at a location. As illustrated in FIG. 3A to FIG. 3C, the cladding on an optical fiber 302 is removed so that the core at which the curve detecting section is to be disposed is exposed. A light absorbing member serving as the curve detecting section 304 is applied to the exposed core. The curve detecting section 304 is disposed at least at a position of a flexible section that changes its shape, such as the inserting section 112 and the connection cable 108. The curve detecting section 304 may not be disposed at a rigid section that does not change its shape, such as the operating section 110. In other words, the curve detecting section 304 may not be disposed at the second shape sensors 208a.

In such a configuration, one part of light that is emitted by the light emitting units 202a and 206a and guided through the optical fiber 302 is absorbed by the light absorbing member serving as the curve detecting section 304 in accordance with the curved state (movable state) of the optical fiber 302. Another part of light is reflected inside the optical fiber 302 and guided to the distal end. The light is then reflected by a reflecting section disposed at the distal end, for example, returned to the optical fiber 302, and received by the light receiving units 202b and 206b. FIG. 3A to FIG. 3C are diagrams illustrating relations between light guided through the optical fiber 302 and the curved state. FIG. 3A illustrates that the optical fiber 302 is curved upward in the drawing, causing a small amount of light to be absorbed, that is, causing a large amount of light to be transmitted. FIG. 3B illustrates that the optical fiber 302 is hardly curved, causing a moderate amount of light to be transmitted. FIG. 3C illustrates that the optical fiber 302 is curved downward in the drawing, causing a large amount of light to be absorbed, that is, causing a small amount of light to be transmitted. Such a relation between the curved state of the optical fiber 302 and the amount of light transmission enables the shape of the optical fiber 302 to be detected based on output from the light receiving units 202b and 206b.

Note that the relation between the curved state of the optical fiber 302 and the amount of light transmission depends on how the curve detecting section 304 is disposed, and is not necessarily such relations as illustrated in FIG. 3A to FIG. 3C.

FIG. 4 is a diagram illustrating a structure in which the optical fibers 302 are installed in the endoscope. In the example of FIG. 4, a bundle of the optical fibers 302 are disposed inside the inserting section 112. To individually detect the curve in the X-axis direction and the curve in the Y-axis direction illustrated in FIG. 4, the optical fibers 302 are disposed in pairs such that the curve detecting section 304 detecting the curve in the X-axis direction are paired with the curve detecting section 304 detecting the curve in the Y-axis direction. Furthermore, the optical fibers 302 are disposed such that pairs of the curve detecting sections 304 are lined along the longitudinal direction (inserting direction) of the inserting section 112. Additionally, the curve detecting sections 304 are lined along the longitudinal direction in such a manner as to detect the shape of the inserting section 112 reaching the vicinity of its distal end.

Inside the inserting section 112, an illumination fiber 306 that guides light for illuminating the interior of the insertion target P from the inserting section 112 and wiring 308 that transmits an imaging signal obtained from the imaging section 112a to the computing unit 1022 in the main body 102 are also disposed.

FIG. 4 illustrates an example of disposing one curve detecting section 304 for each of the optical fibers 302. A plurality of the curve detecting sections 304 may, however, be disposed for each of the optical fibers 302. For example, applying light absorbing members having different wavelength characteristics to different positions on which the curve detecting sections 304 are formed causes the curve detecting sections 304 to individually change the amount of light for the corresponding wavelengths.

The following describes a detecting range of the curve detecting section 304. The curve detecting section 304 illustrated in FIG. 4 detects the curve of the curve detecting section 304 itself. The fact is, however, not that the structure and the material of the inserting section 112 or the connection cable 108, into which the shape sensor is incorporated, causes only the curve detecting section (having a length of 2 mm along the longitudinal direction of the shape sensor, for example) 304 to curve. A shape sensor used in the endoscope usually curves over a certain range (60 mm, for example) in the longitudinal direction. Thus, the curve detecting section 304 may be deemed to actually detect not only the position where it is located but also the curve over a certain range (30 mm each in the inserting direction and the removing direction, 60 mm in total, for example).

Note that specifying a wider detecting range of the curve detecting section 304 decreases the accuracy of detecting the shape. In contrast, narrowing the detecting range increases the accuracy, but also increases the number of the optical fibers 302 and complicates the structure of the shape sensor. It is therefore preferable to specify the range widely to such an extent that it causes no problem in detecting the shape.

The following describes the operation of the insertion apparatus 100.

The operator O holds the operating section 110 and the inserting section 112 and inserts the inserting section 112 into the insertion target P from the entrance Po. The operator presses the insertion target reference position input section 110b once the distal end of the inserting section 112 reaches the entrance Po to the insertion target P. In response to the insertion target reference position input section 110b being pressed, a signal to indicate the timing at which the insertion target reference position input section 110b is pressed is input to the insertion target positional information input unit 1021. The insertion target positional information input unit 1021 acquires a signal from an insertion target reference position input section 110b as association information about the insertion target reference position, and inputs the signal to the computing unit 1022.

The computing unit 1022 performs computations to associate the position of the entrance Po to the insertion target P, which is the position where the distal end of the inserting section 112 is located at the point in time when the insertion target reference position input section 110b is pressed, to be the reference position of the insertion target. To this end, the computing unit 1022 computes the shape of the inserting section 112 from the amount of light transmission detected by the first shape sensor 204 and the amount of light transmission detected by the second shape sensors 208a and 208b (practically, by the second shape sensor 208b). In advance of performing the computation, the relational expression between the change $\Delta$If in the amount of light transmitted by the optical fiber 302 in the shape sensor and the curvature φf of the curve detecting section 304 needs to be obtained. To simplify the description, the relation between the change $\Delta$If in the amount of light transmission and the curvature φf of the curve detecting section 304 is assumed to be expressed by the following function:

$$φf = f (\Delta If).$$

This expression is used to compute the curvature of each of the curve detecting sections 304 from the corresponding amount of light transmission. The shape of the connection cable 108 with respect to the main body 102 serving as the reference unit is then computed from the curvature of each of the curve detecting sections 304 in the first shape sensor 204. In contrast, the shape of the inserting section 112 with respect to the main body 102 serving as the reference unit is computed from the curvature of each of the curve detecting sections 304 in the second shape sensor 208b.

As described above, the rigid section shape memory 1023 stores shape information on the operating section 110. The operating section 110, which is the rigid section, does not change its shape. The shape information can therefore be fixed information. The computing unit 1022 reads the shape information on the operating section 110 stored in the rigid section shape memory 1023, connects the shape of the connection cable 108, the shape of the operating section 110, and the shape of the inserting section 112 with one another to compute the shape of the endoscope as a whole. The computing unit 1022 also computes, on the basis of the shape of the endoscope with respect to the main body 102, which is the reference unit, the position and the direction of the distal end of the inserting section 112 with respect to the main body 102.

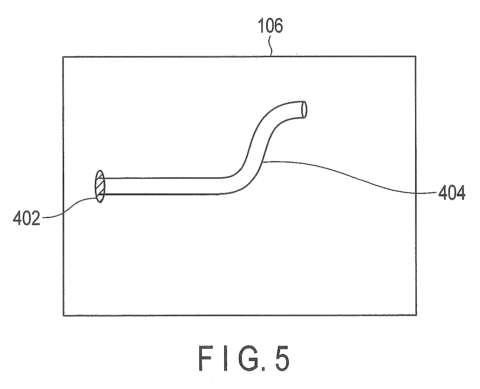

After associating the position of the distal end of the inserting section 112 with the reference position, the computing unit 1022 outputs, to the display 106, information on the shape of the inserting section 112 with respect to the entrance Po to the insertion target P. The display 106 displays an image 404 that shows the shape of the inserting section 112 starting from a reference position 402, as illustrated in FIG. 5, for example. In the present embodiment, the reference position is set at the entrance Po to the insertion target P. As described above, however, the reference position may be determined optionally by the operator O. Even if the reference position is not the entrance Po, the reference position is the position where the distal end of the inserting section 112 is located at the point in time when the operator O presses the insertion target reference position input section 110b. Additionally, the reference position 402 on the display 106 may be configured to be changed. For example, a cursor may be provided on a screen so that the operator O can specify the position with the cursor on the screen as the reference position 402 on display.

In the present embodiment, the position of the distal end of the inserting section 112 is associated with the reference position under the assumption that the insertion target P hardly moves while the endoscope is being used. When the insertion target P moves with respect to the main body 102 serving as the reference unit, the coordinates of the entrance Po to the insertion target P deviates from the position at the time when the insertion target reference position input section 110b is pressed. As a result, the position of the inserting section 112 also deviates with respect to the entrance Po to the insertion target P. Deviation, or movement of the insertion target P, to such an extent that it causes no problem in assisting in operation of the endoscope is acceptable.

As described above, according to the present embodiment, the shape and the position of the inserting section 112 with respect to the main body 102 serving as the reference unit can be detected by detecting the shape of the connection cable 108 from the main body 102 using the first shape sensor 204. This configuration enables the operator O to recognize the shape and the position of the inserting section 112 in the space where the endoscope is used. Consequently, operability is improved.

The shape sensor can be incorporated even into an apparatus that is tubular and has small inner space, such as an endoscope, by using the optical fiber curved shape sensor as the shape sensor. Furthermore, even if the connection cable 108 and the inserting section 112 curve, the shape and the distal end position of the inserting section 112 with respect to the main body 102 serving as the reference unit can be detected by disposing the curve detecting sections 304 in such a manner as to detect the respective shapes of the connection cable 108 and the inserting section 112, which are flexible sections of the endoscope. Additionally, the number of the curve detecting sections 304 can also be reduced by configuring not to detect the shape of the operating section 110, which is the rigid section, thus also causing the number of the optical fibers 302 to be reduced.

In the present embodiment, the insertion target reference position input section is a switch. In such a case, the computing unit 1022 can recognize the reference position of the insertion target P simply by the operator O's pressing the switch when the distal end of the inserting section 112 reaches the entrance Po, which is the reference position at the insertion target P.

In the present embodiment, the computing unit 1022 can also compute the shape and the position of the inserting section 112 with respect to the entrance Po to the insertion target P, which is the reference position. This configuration enables the operator O to operate the operating section 110 while looking at the shape of the inserting section 112 with respect to the entrance Po to the insertion target P, for example. Consequently, the operator O can perform operations easily compared with the case where the operator O operates the operating section 110 while looking at the shape of the inserting section 112 with respect to the main body 102 serving as the reference unit located without regard to the position of the insertion target P.

The operator O can directly look at the entrance Po to the opening of the insertion target P, thus recognizing the position easily. Setting the reference position at the entrance Po to the insertion target P can therefore improve operability.

<Modification of First Embodiment>

In the first embodiment described above, the operator O presses the switch, which is the insertion target reference position input section 110b, so that the computing unit 1022 can determine that the distal end of the inserting section 112 has reached the entrance Po to the insertion target P. The approach through which the computing unit 1022 determines that the distal end of the inserting section 112 has reached the entrance Po to the insertion target P is, however, not limited to switch operation performed by the operator O. For example, the computing unit 1022 may be configured to determine that the distal end of the inserting section 112 has reached the entrance Po to the insertion target P by analyzing an image captured by the imaging section 112a. Examples of approaches to determination using image analysis include determination based on the shape or the color specific to the entrance Po to the insertion target P. Alternatively, determination may be made based on the presence or absence of flicker (When flicker occurs, it is determined that the distal end of the inserting section 112 (i.e. the imaging section 112a) is outside the insertion target P; when the flicker disappears, it is determined that the distal end of the inserting section 112 has reached the entrance Po to the insertion target P) or the presence or absence of linear shapes (If the insertion target is a human body, linear shapes hardly exists inside thereof. Thus, when many linear shapes appear on the image, it is determined that the distal end of the inserting section 112 is outside the insertion target P; when the linear shapes disappear, it is determined that the distal end of the inserting section 112 has reached the entrance Po to the insertion target P).

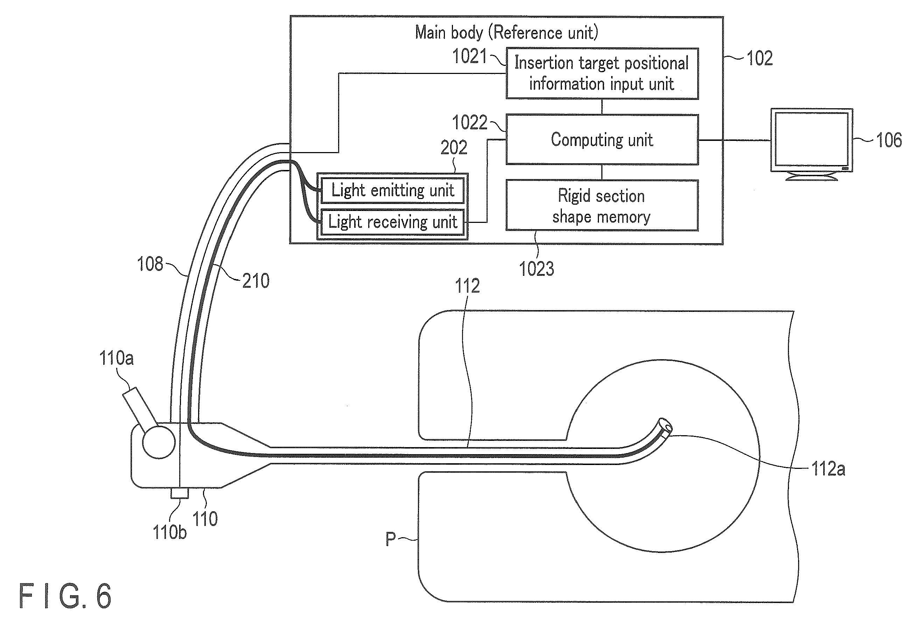

Although the first shape sensor and the second shape sensors are separate in FIG. 2, they may be integrated into a shape sensor 210 as illustrated in FIG. 6. Even in this case, the curve detecting section 304 may not be disposed at the position of the operating section 110.

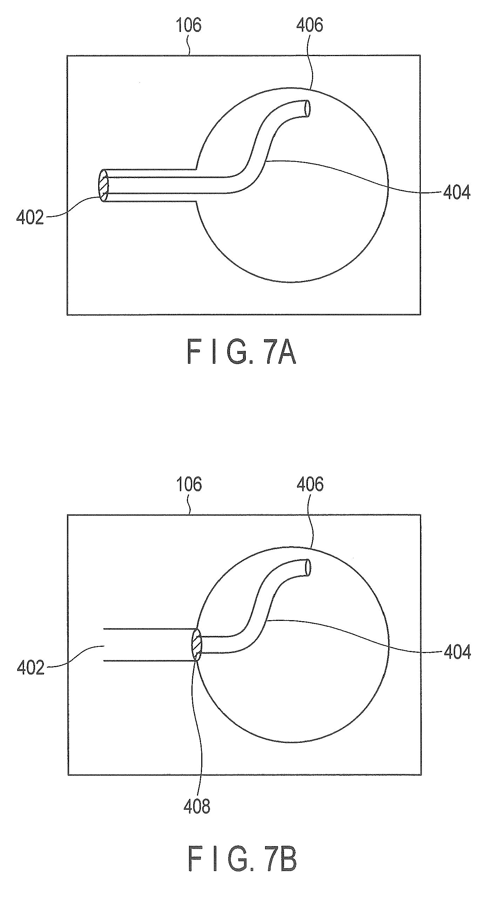

Although the example in which only the shape of the inserting section 112 is displayed on the display 106 is presented above, an image 406 of the interior of the insertion target P may be displayed as well as an image 404 of the shape of the inserting section 112, as illustrated in FIG. 7A. The image of the interior of the insertion target P can be acquired by an X-ray apparatus or a CT apparatus, for example.

The operator O may be allowed to position the reference position of the insertion target P as desired. The operator O performs such positioning by selecting, on the image, the position of an entrance 408 to a shape having space, such as a stomach and a bladder, inside of the insertion target P displayed on the display 106, as illustrated in FIG. 7B, for example. The operator O then presses the insertion target reference position input section 110b once the distal end of the inserting section 112 reaches the entrance to the space previously selected. Subsequent operations are the same as those of the first embodiment described above. The operator O can thus select a desired position as a reference position. Consequently, operability of the endoscope can be further improved. Note that to clarify the difference from FIG. 7A, the shape of the inserting section 112 before being inserted into the reference position (the left of the image 408 of the entrance) is not displayed in FIG. 7B, but may be displayed.

<Second Embodiment>

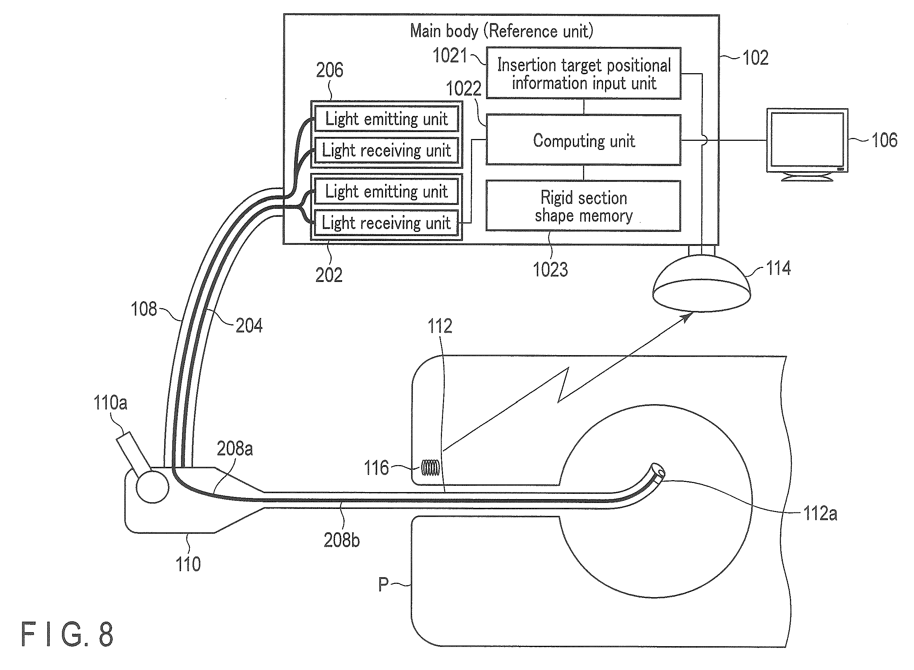

The following describes a second embodiment of the present invention. Differing from the first embodiment, the second embodiment of the present invention includes an insertion target position detector that directly detects the position of the insertion target P. The insertion target position detector in the example of FIG. 8 includes an antenna 114 and a coil 116. The coil 116 is disposed at a reference position of the insertion target P (close to the entrance, for example) and generates a magnetic field. The antenna 114 is disposed at the main body 102, which is the reference unit, and detects changes in the magnetic field generated by the coil 116. The following further describes mainly the points that differ from those of the first embodiment.

[Coil]

The coil 116 serving as a magnetic field transmitter is attached to the insertion target P and generates a magnetic field when a current is passed through the coil 116. The coil 116 is connected electrically to the main body 102, from which a current is received to be driven. Alternatively, the coil 116 is driven cordlessly with a battery mounted therein. The position of the coil 116 installed with respect to the insertion target P needs to be stored in a computing unit 1022 in advance. This configuration enables the computing unit 1022 to recognize the position of the coil 116 as the reference position. The installation position of the coil 116 may be changed at timing other than while the endoscope is being used. In such a case, information on the installation position stored in the computing unit 1022 is also updated. This information may be updated by the operator O.

FIG. 8 illustrates the example of disposing the single coil 116. Practically, a plurality of the coils 116 may be disposed at different positions. Disposing a plurality of coils 116 at different positions can increase the accuracy of detecting the position of the insertion target P.

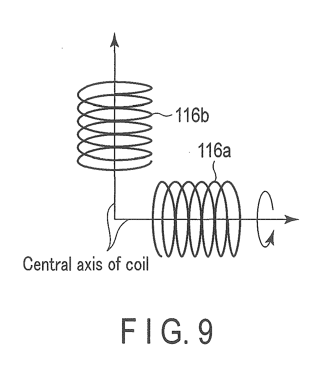

The magnetic field generated by the coil 116 does not change even if the coil 116 rotates about the central axis. Thus, as illustrated in FIG. 9, for example, a plurality of coils 116a and 116b that are different in orientation may be disposed at each location of the insertion target P. For example, the coils 116a and 116b are disposed in such a manner that the central axis of the coil 116a is orthogonal to the central axis of the coil 116b. This configuration enables detection of the reference position even when the insertion target P moves, rotating about the central axis of either coil.

When a plurality of coils are disposed at a location, magnetic fields generated by the respective coils need to be distinguished from each other. A possible approach for this is separation by time, for example. To this end, the coil 116a and the coil 116b are configured to generate magnetic fields in turn.

[Antenna]

The antenna 114 serving as a magnetic field receiver is fixed so as not to change the positional relation with the main body 102. The antenna 114 is connected to an insertion target positional information input unit 1021 and outputs a signal to indicate the intensity and the direction of the magnetic field generated by the coil(s).

The insertion target positional information input unit 1021 acquires the signal from the antenna 114 as association information about the insertion target reference position, and inputs the signal to a computing unit 1022. The computing unit 1022 in the present embodiment stores information about the positional relation between the antenna 114 and the main body 102, and identifies the position of an entrance Po to the insertion target P on the basis of the signal from the insertion target positional information input unit 1021 as well as the information about the positional relation between the antenna 114 and the main body 102.

Although the antenna 114 is fixed so as not to change the positional relation with the main body 102 in the present embodiment, the antenna 114 does not always need to be fixed. As long as the positional relation between the antenna 114 and the main body 102 is known or the positional relation between the antenna 114 and the main body 102 can be detected, the position of the antenna 114 may be changed. For example, if the computing unit 1022 is configured to receive the position of the antenna 114, the position of the antenna 114 may be changed.

In the example of FIG. 8, the coil 116 is disposed on the insertion target P, whereas the antenna 114 is disposed at the main body 102. Contrarily, the antenna 114 may be disposed on the insertion target P, whereas the coil 116 may be disposed at the main body 102.

As described above, according to the present embodiment, the insertion target position detector is included to directly detect the position of the insertion target P. In addition to the advantageous effects same as that of the first embodiment, this configuration avoids a deviation of the shape and the position of an inserting section 112 with respect to the insertion target P, which are computed by the computing unit 1022, even if the insertion target moves.

<Modification of Second Embodiment>

The second embodiment described above presents the insertion target position detector configured to use a magnetic field as an example. Alternatively, the insertion target position detector may be configured to use an electric field or a sound wave. For example, for the configuration using an electric field, an electric field transmitter is disposed at either the insertion target P or the main body 102, and an electric field receiver is disposed at the other. For the configuration using a sound wave, a sound wave transmitter is disposed at either the insertion target P or the main body 102, and a sound wave receiver is disposed at the other.

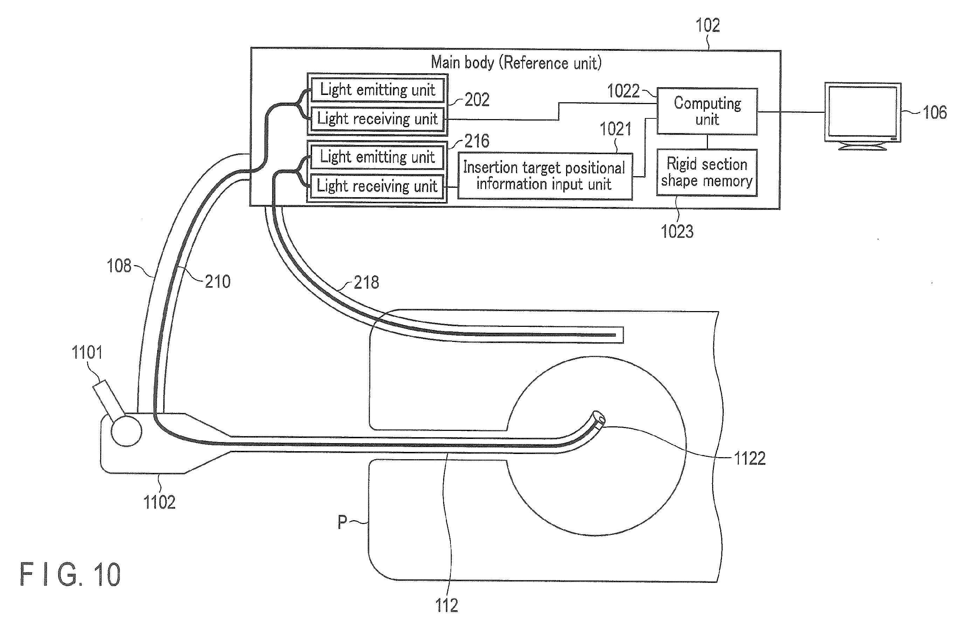

A configuration in which a shape sensor is used as the insertion target position detector (hereinafter, a shape sensor for detecting an insertion target position) is also possible. FIG. 10 is a diagram illustrating a configuration of a flexible endoscope in which the shape sensor for detecting an insertion target position is used. A shape sensor for detecting an insertion target position 218 includes optical fibers, which serves as connecting members for detecting an insertion target position, having curve detecting sections 304 disposed at various locations. The optical fiber has one end connected to a light receiving/emitting unit 216 that is disposed in the main body 102. The one end of the optical fiber is fixed in place and connected to the main body 102 so as not to rotate about the longitudinal axis. The other end of the optical fiber (hereinafter, the distal end of the shape sensor for detecting an insertion target position) is attached to a place of the insertion target P that is stored in a computing unit 1022 in advance, for example, to the vicinity of the entrance Po to the insertion target P. The distal end of the shape sensor for detecting an insertion target position is fixed so as not to change the position and the direction thereof with respect to the insertion target P.

In such a configuration, the computing unit 1022 computes the position and the direction of the distal end of the shape sensor for detecting an insertion target position with respect to the main body 102 in the same manner as computing the position and the direction of the distal end of the inserting section 112. The computing unit 1022 can thus compute the position and the direction of the distal end of the shape sensor for detecting an insertion target position as the position and the direction (attitude) of the insertion target with respect to the main body 102.

When a coil and an antenna are used to detect the position of the insertion target P, coverage of the magnetic field or directions in which the antenna can receive the magnetic field may restrict the detecting range of the position of the insertion target P to be narrow. In contrast to this, when the shape sensor for detecting an insertion target position is used, no antenna needs to be installed. Consequently, the position of the insertion target can be detected within the range covered by the shape sensor for detecting an insertion target position.

<Other Modifications>

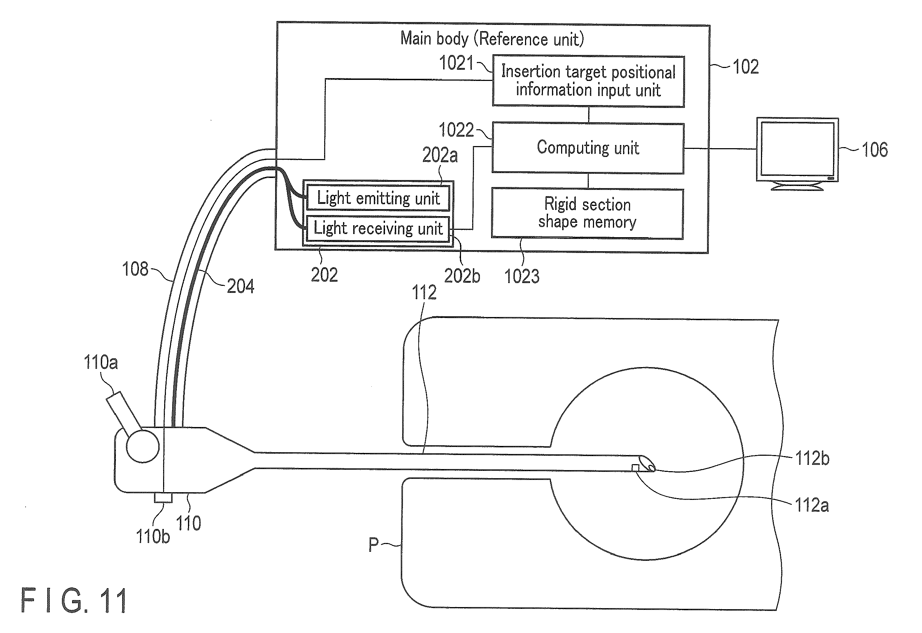

The embodiments and their modifications described above present the flexible endoscope as an example of the insertion apparatus 100. The following describes an exemplary application to a rigid endoscope with reference to FIG. 11. For the rigid endoscope, the shape of the inserting section 112 does not change, eliminating the need for a second shape sensor to be installed. Alternatively, the rigid section shape memory 1023 is configured to store shape information on the inserting section 112 (information on the length, information on the attachment direction of the inserting section 112 with respect to the operating section 110, the direction of an opening 112b of the imaging section 112a with respect to the inserting section 112, for example), in addition to shape information on the operating section 110. The computing unit 1022 reads the respective shape information on the operating section 110 and the inserting section 112 stored in the rigid section shape memory 1023. Subsequently, the computing unit 1022 connects the shape of the connection cable 108 from the main body 102 serving as the reference unit, detected by the first shape sensor 204, the shape of the operating section 110 stored in the rigid section shape memory 1023, and the shape of the inserting section 112 with one another to compute the shape of the endoscope as a whole. This configuration enables the operator O to grasp the extent to which the inserting section 112 is inserted into the insertion target P. Consequently, operability is improved.

For the rigid endoscope, the opening 112b for the imaging section 112a may be formed in a slanted manner as illustrated in FIG. 11. In such a case, rotation of the inserting section 112 about the inserting direction thereof can vary the direction of the opening 112b, that is, a site to be observed. In the present modification, the first shape sensor 204 can detect the direction of the end of the connection cable 108 on the operating section 110, and thus can also detect the rotation direction of the operating section 110 with respect to the insertion target P, that is, the rotation direction of the distal end of the inserting section 112. Additionally, the direction of the opening 112b is also stored as the shape information on the inserting section 112. Consequently, the direction of the opening 112b with respect to the insertion target P can also be detected, enabling the operator O to find out the direction of observation with respect to the insertion target P.

FIG. 11 illustrates the example corresponding to the first embodiment. However, the techniques similar to those illustrated in FIG. 11 can be employed to the modification of the first embodiment, and the second embodiment and its modification.

Although the present invention has been described based on the embodiments, the present invention is not limited to the foregoing embodiments, and a variety of modifications and applications can be made within the scope of the spirit of the present invention as a matter of course.

**Claims**

1.   An insertion apparatus comprising:

an inserting section that is to be inserted into an insertion target;
a reference unit that is disposed outside the insertion target;
a connecting member that includes a movable section configured to move continuously and connects the inserting section and the reference unit; and
a first shape sensor that detects a shape of the connecting member with respect to the reference unit by detecting a movable amount of the connecting member.

2. The insertion apparatus according to claim 1, wherein
the inserting section is configured to move continuously, and
the insertion apparatus further comprises a second shape sensor that detects a shape of the inserting section with respect to the reference unit by detecting a movable amount of the inserting section.

3. The insertion apparatus according to claim 2, further comprising:

an insertion target positional information input unit that receives insertion target positional information concerning a relative position between the reference unit and the insertion target; and
a computing unit that computes a shape of the inserting section for the insertion target using the shape of the connecting member detected by the first shape sensor, the shape of the inserting section detected by the second shape sensor, and the insertion target positional information.

4. The insertion apparatus according to claim 2, wherein the second shape sensor is an optical fiber curved shape sensor comprising:

a light emitting unit;
an optical fiber that guides light from the light emitting unit and changes optical characteristics of the guided light in accordance with a curved shape of the inserting section; and
a light receiving unit that receives the light guided by the optical fiber.

5. The insertion apparatus according to claim 1, wherein
the inserting section is a rigid section that is immovable, and
the insertion apparatus further comprises a rigid section shape memory that stores shape information on the inserting section.

6. The insertion apparatus according to claim 5, further comprising:

an insertion target positional information input unit that receives insertion target positional information concerning a relative position between the reference unit and the insertion target; and
a computing unit that computes a shape of the inserting section for the insertion target using the shape of the connecting member detected by the first shape sensor, the shape information on the inserting section stored in the rigid section shape memory, and the insertion target positional information.

7. The insertion apparatus according to claim 3, wherein
the insertion target positional information is association information to compute a reference position set for the insertion target based on a position of a distal end of the inserting section, and
the computing unit computes the reference position in accordance with the association information.

8. The insertion apparatus according to claim 7, wherein
the association information is information to indicate timing at which the distal end of the inserting section reaches the reference position, and
the computing unit computes the reference position based on the shape of the connecting member and the shape of the inserting section that are computed at timing when the distal end of the inserting section reaches the reference position.

9. The insertion apparatus according to claim 8, wherein the insertion target positional information input unit inputs, to the computing unit, a signal input by an insertion target reference position input section that is operated by an operator at timing when the distal end of the inserting section reaches the reference position.

10. The insertion apparatus according to claim 7, wherein
the insertion apparatus includes an imaging section, the association information is an image captured by the imaging section, and
the computing unit determines timing when the distal end of the inserting section reaches the reference position, based on the image captured by the imaging section.

11. The insertion apparatus according to claim 7, wherein the reference position is an entrance to an opening of the insertion target.

12. The insertion apparatus according to claim 3, wherein the insertion target positional information is information on a relative position between the reference unit and the reference position input by an insertion target position detector that detects the relative position between the reference unit and the reference position set for the insertion target.

13. The insertion apparatus according to claim 12, wherein
the insertion target position detector comprises:

a transmitter that transmits at least one of a magnetic field, an electric field, and a sound wave; and

a receiver that receives the magnetic field, the electric field, and the sound wave transmitted by the transmitter,

wherein one of the transmitter and the receiver is disposed at the insertion target,

the other of the transmitter and the receiver is disposed so as to maintain a relative position with the reference position at least while the inserting section is being used, and

a relative position between the reference position and the insertion target is computed from a change in the magnetic field, the electric field, and the sound wave received by the receiver.

14. The insertion apparatus according to claim 12, wherein

the insertion target position detector is an insertion target position detection shape sensor that includes an insertion target position detector connecting member configured to connect from the reference position to the insertion target, and detects a curve of the insertion target position detector connecting member to detect a shape of the insertion target position detector connecting member, and

the computing unit computes a position of the insertion target with respect to the reference position, based on the shape of the insertion target position detector connecting member detected by the insertion target position detection shape sensor.

15. The insertion apparatus according to claim 14, wherein

the insertion target position detection shape sensor is an optical fiber curved shape sensor comprising:

a light emitting unit;

an optical fiber that guides light from the light emitting unit and changes optical characteristics of the guided light in accordance with a curved shape of the inserting section; and

a light receiving unit that receives the light guided by the optical fiber.

16. The insertion apparatus according to claim 3, wherein

the connecting member further includes a rigid section that is connected to the movable section,

the insertion apparatus further comprises a rigid section shape memory that stores shape information on the rigid section of the connecting member,

the first shape sensor detects at least a shape of the movable section as a shape of the connecting member, and

the computing unit further includes a sub-computing unit that computes at least one of a shape of the inserting section with respect to the reference unit and a shape of the inserting section for the insertion target using shape information on the rigid section of the connecting member.

17. The insertion apparatus according to claim 1, wherein

the first shape sensor is an optical fiber curved shape sensor comprising:

a light emitting unit;

an optical fiber that guides light from the light emitting unit and changes optical characteristics of the guided light in accordance with a curved shape of the inserting section; and

a light receiving unit that receives the light guided by the optical fiber.

FIG. 1

FIG.2

Main body (Reference unit) — 102

206 206a — 206b

Insertion target positional information input unit — 1021

Light emitting unit

Light receiving unit — 206

Light emitting unit

Light receiving unit — 202 202b 202a

Computing unit — 1022

Rigid section shape memory — 1023

106

108 — 204

110a

208a

110b — 110

208b

112

202a

P

112a

EP 2 979 612 A1

F I G. 3A

F I G. 3B

F I G. 3C

F I G. 4

FIG. 5

FIG.6

Main body (Reference unit)

1021 — Insertion target positional information input unit — 102

1022 — Computing unit

202

Light emitting unit

Light receiving unit

Rigid section shape memory

1023

106

108 210

110a

110b 110

112

112a

P

EP 2 979 612 A1

F I G. 7A

F I G. 7B

FIG. 8

116b

116a

Central axis of coil

F I G. 9

Main body (Reference unit)

102

106

Computing unit — 1022

Rigid section shape memory — 1023

1021

Insertion target positional information input unit

202 — Light emitting unit / Light receiving unit

216 — Light emitting unit / Light receiving unit

218

210

108

1101

1102

1122

112

P

F I G. 10

Main body (Reference unit)

1021 — Insertion target positional information input unit

1022 — Computing unit

202a — Light emitting unit

Light receiving unit

Rigid section shape memory

102

106

108

204

110a

110b  110

202  202b  1023

112

P

112b

112a

FIG. 11

EP 2 979 612 A1

## INTERNATIONAL SEARCH REPORT

| International application No. |
| --- |
| PCT/JP2014/057484 |

| A. CLASSIFICATION OF SUBJECT MATTER |
| --- |
| *A61B1/00*(2006.01)i, *G02B23/24*(2006.01)i |

According to International Patent Classification (IPC) or to both national classification and IPC

| B. FIELDS SEARCHED |
| --- |
| Minimum documentation searched (classification system followed by classification symbols)<br>A61B1/00, G02B23/24 |

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
| --- | --- | --- |
| X<br>A | WO 2010/050526 A1 (Olympus Medical Systems Corp.),<br>06 May 2010 (06.05.2010),<br>paragraphs [0071] to [0078]; fig. 20<br>& US 2011/0098533 A1 & JP 4759654 B<br>& EP 2351509 A1 & CN 102196761 A | 1–2,4,17<br>3,5–16 |
| X<br>A | JP 2011-56280 A (Olympus Corp.),<br>24 March 2011 (24.03.2011),<br>claim 1; paragraph [0019]; fig. 1, 4<br>(Family: none) | 1–2<br>3–17 |

| [×] Further documents are listed in the continuation of Box C. | [ ] See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 16 April, 2014 (16.04.14) | 28 April, 2014 (28.04.14) |

| Name and mailing address of the ISA/<br>Japanese Patent Office | Authorized officer |
| --- | --- |
| Facsimile No. | Telephone No. |

Form PCT/ISA/210 (second sheet) (July 2009)

**INTERNATIONAL SEARCH REPORT**

International application No.

PCT/JP2014/057484

C (Continuation). DOCUMENTS CONSIDERED TO BE RELEVANT

| Category* | Citation of document, with indication, where appropriate, of the relevant passages | Relevant to claim No. |
|---|---|---|
| A | Microfilm of the specification and drawings annexed to the request of Japanese Utility Model Application No. 4431/1991(Laid-open No. 95002/1992) (Tadashi HATANO), 18 August 1992 (18.08.1992), abstract; paragraphs [0004], [0012] & US 5228429 A & EP 495351 A1 & DE 69206827 C & DE 69206827 D | 1-17 |

Form PCT/ISA/210 (continuation of second sheet) (July 2009)

**EP 2 979 612 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003052614 A